**Europäisches Patentamt**

**European Patent Office**

**Office européen des brevets**

(19)

(11) Publication number : **0 403 304 B1**

# (12) EUROPEAN PATENT SPECIFICATION

(45) Date of publication of patent specification :
**02.03.94 Bulletin 94/09**

(51) Int. Cl.⁵ : **A61K 7/06, A61K 7/08**

(21) Application number : **90306564.7**

(22) Date of filing : **15.06.90**

(54) **Shampoo composition.**

(30) Priority : **16.06.89 GB 8913880**

(43) Date of publication of application :
**19.12.90 Bulletin 90/51**

(45) Publication of the grant of the patent :
**02.03.94 Bulletin 94/09**

(84) Designated Contracting States :
**AT BE CH DE DK ES FR GB GR IT LI NL SE**

(56) References cited :
**EP-A- 0 007 785**
**EP-A- 0 018 717**
**EP-A- 0 086 070**
**EP-A- 0 232 982**
**DE-A- 2 401 752**
**CHEMICAL ABSTRACTS, vol. 110, no. 12, 20 March 1989 Columbus, Ohio, USA J.C. Hill et al.: "The skin plasticization effect of a medium chain 2-hydroxy acid and the use of potentiators" page 406; column 1; ref. no. 101521R & J. Appl. Cosmetol. 1988,6(2),pages 53-68**

(56) References cited :
**CHEMICAL ABSTRACTS, vol. 101, no. 2, 9 July 1984 Columbus, Ohio, USA Susan G. Alderson et al.: "Effect of 2-hydroxyacids on guinea pig footpad stratum corneum: mechanical properties and binding studies" page 288; column 2; ref. no. 12004W & Int. J. Cosmet. Sci. 1984,6(2),pages 91-100**

(73) Proprietor : **UNILEVER PLC**
**Unilever House Blackfriars**
**London EC4P 4BQ (GB)**

(84) **GB**
Proprietor : **UNILEVER N.V.**
**Weena 455**
**NL-3013 AL Rotterdam (NL)**

(84) **BE CH DE DK ES FR GR IT LI NL SE AT**

(72) Inventor : **Gallagher, Peter**
**1 Mission Cottage, (of Moss Lane)**
**Burscough, Lancashire (GB)**
Inventor : **McGee, Thomas**
**74 Stanley Road**
**Hoylake, Wirral, Merseyside L47 LH2 (GB)**

(74) Representative : **Tonge, Robert James**
**UNILEVER PLC Patent Division Colworth House Sharnbrook**
**Bedford MK44 1LQ (GB)**

EP 0 403 304 B1

## Description

FIELD OF INVENTION

The invention relates to shampoo compositions containing 2-hydroxyalkanoic acids.

BACKGROUND OF THE INVENTION

Certain 2-hydroxyalkanoic acids are known for their skin benefits when included in compositions for topical application to the skin. Such benefits include both increased extensibility and improved appearance.

Hair which has reduced elasticity, as a result of ageing or of chemical or mechanical treatment regimes is more likely to fracture when subjected to mechanical stress. By increasing hair elasticity, the hair becomes not only more resistant to such stress, but also more appealing cosmetically. The hair feels softer and is less flyaway.

Hydroxycarboxylic acids have previously been included in skin creams where they have been shown to give some benefit in the treatment of various skin disorders. Examples of such skin creams can be found in US 3 920 835, US 3 984 566 or US 4 363 815, all in the names of Van Scott and Yu.

Skin creams containing a combination of 2-hydroxyoctanoic acid and alkyl lactate are disclosed in US 4 507 319 (Unilver), and are said to be particularly effective against acne.

EP 7785 (Unilever) relates to skin compositions containing hydroxylated $C_6$ to $C_{10}$ carboxylic acids which can be formulated with a number of vehicles including anionic emulsifiers. These compositions contain no co-acid buffer to maintain an acid pH.

The use of 2-hydroxy octanoic acid in the treatment of dandruff and of excessively dry scalp with defective hair growth is disclosed in EP 232 982 (Unilever), in which the acid is applied to the hair in a composition which also comprises nonionic surfactant. Nonionic surfactant is preferred in those applications to minimise exacerbation of the skin conditions.

DE 2 110 993 (Henkel) relates to liquid cleaning compositions which comprise alkali metal salts of hydroxycarboxylic acids. These compositions contain no co-acid butter to maintain an acid pH.

It has now been found surprisingly that when certain 2-hydroxyalkanoic acids are incorporated into shampoo compositions, they increase the elasticity of the hair, thus producing a cosmetic benefit. This effect builds up over subsequent applications of the 2-hydroxyalkanoic acid.

The effect can be seen on application of 2 hydroxy alkanoic acids of chain length $C_4$ to $C_{20}$.

BRIEF SUMMARY OF THE INVENTION

The invention provides a method of enhancing the elasticity of hair comprising applying thereto a shampoo composition comprising:

(a) from 0.1 to 20% by weight of a 2-hydroxyalkanoic acid chosen from 2-hydroxyhexanoic acid, 2-hydroxyoctanoic acid, 2-hydroxydecanoic acid or mixtures thereof;

(b) from 2 to 40% by weight of surfactant. Selected from anionic, amphoteric and/or zwitterionic surfactants; and

(c) from 0.1 to 10% by weight of a co-acid buffering agent.

A minor proportion of the surfactant might be a co-surfactant from another class such as a nonionic surfactant.

It is particularly envisaged that forms of the invention will have 2 to 40% anionic surfactant, possibly accompanied by other surfactant.

The invention provides, in another aspect, the use of the above-defined compositions for enhancing the elasticity of hair.

DETAILED DESCRIPTION OF THE INVENTION

The 2-hydroxyalkanoic acid

The shampoo composition of the invention contains from 0.1 to 20% by weight, preferably from 1 to 10% by weight of a 2-hydroxyalkanoic acid, chosen from 2-hydroxyhexanoic acid, 2-hydroxyoctanoic acid, 2-hydroxydecanoic acid or mixtures thereof. The most preferred acid is 2-hydroxyoctanoic acid.

If less than 0.1% by weight of 2-hydroxyalkanoic acid is included in the composition, no increase in elasticity of the hair is observed, and if more than 20% by weight is included, no added benefit is seen.

## Surfactant

The shampoo composition of the invention also comprises from 2 to 40% by weight, preferably 5 to 20% by weight, of surfactant, all or a majority of which is anionic, amphoteric or zwitterionic. In particular there may be at least 2% of anionic (so that the quantity of anionic is in the range 2 to 40%) possibly accompanied by surfactant from another class. Anionic surfactant may constitute the majority proportion of the surfactant present.

An anionic surfactant is preferably chosen from acyl glutamates, acyl peptides, sarcosinates, ester carboxylic acids, acyl isethionates, $\alpha$-olefin sulphonates, sulphosuccinates, alkyl benzene sulphonates, amides of N-methyl taurine, $\alpha$-sulpho fatty acids, alkyl sulphates such as sodium, magnesium or ammonium lauryl sulphate and alkyl ether sulphates, and is most preferably sodium lauryl ether sulphate (2EO).

Amphoteric and zwitterionic surfactants which may be used, especially as a co-surfactant in conjunction with anionic surfactant, include alkyl betaines, alkyl amido propyl betaines, alkyl ampho glycinates and sulphobetaines (sultaines).

Nonionic surfactants which may be present, but only as a minor proportion of the surfactant mixture, include ethoxylates for example of alcohols, sugar esters, esters of glycols, esters of glycerol, ethoxylated sorbitan esters and amine oxides.

## Co-acid buffering agent

The composition also comprises a co-acid buffering agent which acts to maintain an acid pH. We have found that the hair can buffer a weak solution of 2-hydroxyalkanoic acid giving rise to the salt of the acid, which has a lower penetration than the acid itself.

Suitable co-acid buffering agents include lactic acid, citric acid, tartaric acid, acetic acid, formic acid, malonic acid, glycolic acid, thioglycollic acid, benzoic acid, adipic acid, malic acid and mesaconic acid. The most preferred acid is lactic acid.

The co-acid buffering agent is present in the composition in an amount of 0.1 to 10%, preferably at least 1%, more preferably from 2 to 5% by weight. The total of 2-hydroxyalkanoic acid buffering agent will frequently be at least 1.7% better at least 3 or 5% by weight of the composition.

The pH of the composition is preferably in the range of from 3 to 5.

## Other ingredients

The shampoo composition of the invention may also further comprise a deposition agent which aids deposition of the 2-hydroxy alkanoic acid onto the hair.

Suitable deposition agents include the cationic cellulose ethers described in US Patent Nos. 3 816 616 and 4 272 515 and which are available commercially from Union Carbide Corporation as Polymer JR®. Polymer JR has the CTFA designation Polyquaternium 10. Other suitable materials are the cationic polygalactomannan gum deriviatives described in US Patent No. 4 298 494 which are commercially available under the trade mark Jaguar from Celanese-Stein Hall. An example of a suitable material has the CTFA designation guar hydroxypropyltrimonium chloride and is available under the name Jaguar C13S. Other suitable materials include that known as Jaguar C17 and Jaguar C16 which is hydroxypropylated cationic guar derivative containing hydroxypropyl substituent groups as well as cationic quaternary ammonium groups.

Other deposition agents useful in the shampoos of the present invention include cationic polyamide polymers such as the low molecular weight adipic acid/diethylene-triamine polyamide and the copolymers of vinylpyrrolidone and dimethylaminoethyl methacrylate quaternised with dimethyl sulphate (Gafquat® 755, GAF Corporation) described in US Patent No. 4 080 310; the graft cationic copolymer containing N-vinylpyrrolidone, dimethylamonoethyl methacrylate and polyethylene glycol described in US Patent No. 048 301; the mineral acid salts of the amono-alkyl esters of homo- and copolymers of unsaturated carboxylic acids having from 3 to 5 carbon atoms cescribed in US Patent No. 4 009 256; and the polymers of etherified starch described in US Patent No. 3 186 911.

The high molecular weight polymers sold under the trade mark Merquat by Merck & Co. Inc., are also suitable for use as deposition agents in the present shampoos. Representative ones are Merquat 100, a highly charged cationic dimethyldiallylammonium chloride homopolymer, and Merquat 550, a highly charged cationic copolymer prepared with dimethyldiallylammonium chloride and acrylamide. These materials are designated in the CFTA dictionary as Quaternium-40 and Quaternium-41, respectively.

The shampoo composition of the invention comprises also certain ingredients known in the art and necessary to the particular formulation. Examples of other ingredients include surfactants, viscosity control agents, solubility control agents, foam boosters, opacifiers, perfumes, colouring agents, conditioning agents such as silicones, preservatives, proteins, polymers, buffearing agents and water.

PROCESS

The shampoo composition of the invention is formulated by mixing together the required ingredients in the amounts specified.

PRODUCT FORMS AND PACKAGING

The shampoo composition of the invention is commonly packaged in a bottle with lid or a dispenser with a pump. In General, the composition of the invention may be formulated in any manner that allows application to the hair so that the benefit conferred by the 2-hydroxyalkanoic acid on the hair is discernible. possible packaging variants are those known to the man skilled in the field of hair treatment compositions.

ADVANTAGES OF THE INVENTION

The shampoo composition of the invention has been shown in tests involving Instron measurements of hair elasticity to increase the elasticity of hair significantly at the 95% confidence level. Although applicant does not wish to be bound by theory, it is thought that there exists a correlation between the increase in elasticity of hair when treated with compositions of the invention and the degree of plasticisation of the hair protein chains. Pulsed NMR measurements confirm that plasticisation in hair treated with a composition containing a 2-hydroxyalkanoic acid is greater than in untreated hair. The effect is particularly marked in the case of hair that has been damaged chemically or mechanically.

Hair with increased elasticity tends to break less easily under mechanical stress such as brushing or combing. Negroid hair that has been treated with the composition of the invention becomes softer, more pliable, and more fracture-resistant than untreated hair. European and Thai hair show improved elasticity.

COMPARATIVE EXAMPLES

The following comparative examples illustrate the effect of 2-hydroxyalkanoic acid retention on hair properties.

Comparative Example 1

Hair switches were immersed in a 1% by weight aqueous solution of 2-hydroxyoctanoic acid (pH 3.0) for 24 hours. The hair: liquor ratio was 1:200 by weight. Instron tests showed that the increase in elasticity resulting from treatment with the 2-hydroxyoctanoic acid was statistically significant at the 95% confidence level as compared to virgin, untreated hair.

Comparative Example 2

Switches of Thai hair were soaked for 24 hours in 0.0625M solutions of 2-hydroxy alkanoic acids having chain lengths of $C_2$, $C_6$, $C_8$ and $C_{14}$, and in a hair to liquor ratio of 1:200. The $C_{14}$ hydroxy acid is sparingly soluble in water so an ethanolic solution was used. Control results were obtained by soaking hair switches in water or ethanol (as control for $C_{14}$) for 24 hours.

It was found that all of the acids tested increase the elasticity of the hair with the 2-hydroxy octanoic acid giving the optimum effect.

Comparative Example 3

This comparative example illustrates the effect of a co-acid buffer on retention of 2-hydroxyalkanoic acid.

Hair switches were treated with a composition containing 1% radiolabelled 2-hydroxyoctanoic acid and 2% lactic acid at a pH of 3.0. After several applications, the degree of retention of the 2-hydroxyoctanoic acid by the hair was measured and was found to be greater than for hair treated with a similar composition containing no lactic acid.

This demonstrates that 2-hydroxyalkanoic acid retention by the hair is enhanced in the presence of a co-acid buffer.

Comparative Example 4

Hair switches (0.5g, 10 cm long) were washed. A test composition, containing varying levels of 2-hydroxyoctanoic acid and lactic acid as set out below, was applied to each switch, left for one minute, and the switch was rinsed for 30 seconds. The 2-hydroxyoctanoic acid was radiolabelled with $^{14}$C so that the amount of 2-hydroxyoctanoic acid retained on the hair could be assesed.

The switch was blown dry, shampooed with a different composition (lacking 2-hydroxyalkanoic acid) and the test composition reapplied. The level of radioactivity retained on the hair was measured after 1, 5 and 10 such treatments.

The test compositions all had a pH of 3 and contained:-

A. 1% by weight 2-hydroxyoctanoic acid

B. 1% by weight 2-hydroxyoctanoic acid and 2% by weight lactic acid

C. 2% by weight 2-hydroxyoctanoic acid and 4% by weight lactic acid

The results were as follows. The measurements are given as g of 2-hydroxyoctanoic acid ($\times 10^{-4}$) retained per g of hair.

|  |  |  | g/g hair ($\times 10^{-4}$) |
|---|---|---|---|
| No. applications | A | B | C |
| 1 | 2.15 | 3.03 | - |
| 5 | 3.12 | 7.55 | 16.5 |
| 10 | 4.08 | 11.1 | - |

It can be seen that the level of 2-hydroxyoctanoic acid which is retained on the hair is significantly enhanced by the addition of lactic acid.

The following Examples illustrates shampoo compositions according to the invention. The ingredients are mixed together to form the shampoo, and the pH of each of the compositions is adjusted to 3.0 to 5.0 using base.

Example 1

|  | % wt |
|---|---|
| SLES 2EO | 9.0 |
| Empilan CDE (1) | 2.0 |
| Hydroxyoctanoic acid | 5.0 |
| Lactic acid | 1.0 |
| Colour, perfume, preservative | qs |
| Water | to 100 |

(1) Empilan® CDE is cocodiethanolamide

Example 2

|  | %wt |
|---|---|
| Dialkylsulphosuccinate | 9.5 |
| Urea | 15.0 |
| Hydroxyhexanoic acid | 5.5 |
| Acetic acid | 1.7 |
| Butane-1, 3-diol | 2.5 |
| Water | to 100 |

Example 3

|  | %wt |
|---|---|
| Alpha-olefin sulphonate | 8.5 |
| Glucamate DOE 120 (2) | 4.5 |
| Hydroxydecanoic acid | 0.4 |
| Lactic acid | 1.3 |
| Colour, perfume, preservative | qs |
| Water | to 100 |

(2) Glutamate DOE 120 is ethoxylated methyl glucoside dioleate.

Example 4

|  | %wt |
|---|---|
| DOBS 102 (3) | 10.0 |
| Glucamate DOE 120 | 4.5 |
| Hydroxyoctanoic acid | 3.0 |
| Lactic acid | 2.0 |
| Colour, perfume, preservative | qs |
| Water | to 100 |

(3) DOBS 102 is $C_{12}$-$C_{14}$ alkyl benzene sulphonate

**Claims**

1. A method of enhancing the elasticity of hair comprising applying thereto an aqueous shampoo composition comprising:
   (a) from 0.1 to 20% by weight of a 2-hydroxyalkanoic acid chosen from 2-hydroxyhexanoic acid, 2-hydroxyoctanoic acid, 2-hydroxydecanoic acid or mixtures thereof;
   (b) from 2 to 40% by weight of surfactant selected from anionic, amphoteric and/or zwitterionic surfactants; and
   (c) from 0.1 to 10% by weight of a co-acid buffering agent.

2. A method according to Claim 1, wherein the 2-hydroxyalkanoic acid in the composition is 2-hydroxyoctanoic acid.

3. A method according to Claim 1 or Claim 2, wherein the 2-hydroxyalkanoic acid in the composition is present in an amount from 1 to 10% by weight.

4. A method according to any one of claims 1 to 3, wherein the composition contains 2 to 40% by weight anionic surfactant.

5. A method according to claim 4, wherein the anionic surfactant is chosen from acyl glutamates, acyl peptides, sarcosinates, ester carboxylic acids, acyl isethionates, $\alpha$-olefin sulphonates, sulphosuccinates, alkyl sulphates, alkyl ether sulphates, alkyl aryl sulphonates, amides of N-methyl taurine or $\alpha$-sulpho fatty acids.

6. A method according to Claim 4, wherein the anionic surfactant is chosen from sodium lauryl ether sulphate 2EO or sodium lauryl sulphate 3EO.

7. A method according to any preceding Claim, wherein the co-acid buffering agent in the composition is lactic acid.

8. A method according to any preceding Claim, wherein the co-acid buffering agent in the composition is present in an amount from 2 to 5% by weight.

9. A method according to any preceding Claim, wherein the composition further comprises a deposition agent in an amount from 0.01 to 5% by weight.

10. A method according to Claim 9, wherein the deposition agent is chosen from Polyquaternium 10, Quaternium 40, Quaternium 41 and guar hydroxypropyltrimonium chloride.

11. A method according to Claim 9, wherein the deposition agent in the composition is present in an amount from 0.5 to 3% by weight.

12. A method according to any preceding Claim, wherein the total of 2-hydroxyalkanoic acid and co-acid buffering agent in the composition is at least 3% by weight of the composition.

13. Use for enhancing the elasticity of hair of an aqueous shampoo which comprises:
    (a) from 0.1 to 20% by weight of a 2-hydroxyalkanoic acid chosen from 2-hydroxyhexanoic acid, 2-hydroxyoctanoic acid, 2-hydroxydecanoic acid or mixtures thereof;
    (b) from 2 to 40% by weight of surfactant selected from anionic, amphoteric and/or zwitterionic surfactants; and
    (c) from 0.1 to 10% by weight of a co-acid buffering agent.

**Patentansprüche**

1. Verfahren zur Steigerung der Elastizität von Haar, wobei eine wäßrige Shampoo-Zusammensetzung darauf aufgetragen wird, enthaltend:
   (a) 0.1 bis 20 Gew.% einer 2-Hydroxyalkansäure, ausgewählt aus 2-Hydroxyhexansäure, 2-Hydroxyoctansäure, 2-Hydroxydecansäure oder Mischungen davon;
   (b) 2 bis 40 Gew.% Tensid, ausgewählt aus anionischen, amphoteren und/oder zwitterionischen Tensiden; und

(c) 0.1 bis 10 Gew.% eines Zusatzsäurepuffers.

2. Verfahren nach Anspruch 1, worin die 2-Hydroxyalkansäure in der Zusammensetzung 2-Hydroxyoctansäure ist.

3. Verfahren nach Anspruch 1 oder Anspruch 2, worin die 2-Hydroxyalkansäure in der Zusammensetzung in einer Menge von 1 bis 10 Gew.% vorhanden ist.

4. Verfahren nach einem der Ansprüche 1 bis 3, worin die Zusammensetzung 2 bis 40 Gew.% anionisches Tensid enthält.

5. Verfahren nach Anspruch 4, worin das anionische Tensid ausgewählt wird aus Acylglutamaten, Acylpeptiden, Sarcosinaten, Estercarbonsäuren, Acylisethionaten, $\alpha$-Olefinsulfonaten, Sulfosuccinaten, Alkylsulfaten, Alkylethersulfaten, Alkylarylsulfonaten, Amiden von N-Methyltaurin oder $\alpha$-Sulfofettsäuren.

6. Verfahren nach Anspruch 4, worin das anionische Tensid ausgewählt ist aus Natrium-laurylethersulfat (2EO) oder Natriumlaurylsulfat 3EO.

7. Verfahren nach einem der vorhergehenden Ansprüche, worin das Zusatzsäurepuffermittel in der Zusammensetzung Milchsäure ist.

8. Verfahren nach einem der vorhergehenden Ansprüche, worin das Zusatzsäurepuffermittel in der Zusammensetzung in einer Menge von 2 bis 5 Gew.% vorhanden ist.

9. Verfahren nach einem der vorhergehenden Ansprüche, worin die Zusammensetzung zusätzlich ein Ablagerungsmittel in einer Menge von 0.01 bis 5 Gew.% enthält.

10. Verfahren nach Anspruch 9, worin das Ablagerungsmittel ausgewählt ist aus Polyquaternium 10, Quaternium 40, Quaternium 41 und Guar-hydroxypropyltrimonium-chlorid.

11. Verfahren nach Anspruch 9, worin das Ablagerungsmittel in der Zusammensetzung in einer Menge von 0.5 bis 3 Gew.% vorhanden ist.

12. Verfahren nach einem der vorhergehenden Ansprüche, worin das Total von 2-Hydroxyalkansäure und Zusatzsäurepuffermittel in der Zusammensetzung mindestens 3% des Gewichts der Zusammensetzung beträgt.

13. Verwendung eines wäßrigen Shampoos zur Steigerung der Haarelastizität, enthaltend:

(a) 0.1 bis 20 Gew.% einer 2-Hydroxyalkansäure, ausgewählt aus 2-Hydroxyhexansäure, 2-Hydroxyoctansäure, 2-Hydroxydecansäure oder Mischungen davon;

(b) 2 bis 40 Gew.% Tensid, ausgewählt aus anionischen, amphoteren und/oder zwitterionischen Tensiden; und

(c) 0.1 bis 10 Gew.% eines Zusatzsäurepuffers.

**Revendications**

1. Procédé pour favoriser l'élasticité du cheveu comprenant l'application sur celui-ci d'une composition de shampooing aqueuse comprenant:

(a) de 0,1 à 20% en poids d'un acide 2-hydroxyalcanoïque choisi parmi l'acide 2-hydroxyhexanoïque, l'acide 2-hydroxyoctanoïque, l'acide 2-hydroxydécanoïque ou leurs mélanges;

(b) de 2 à 40% en poids de tensio-actif, choisi parmi les tensio-actifs anioniques, amphotériques et/ou zwittérioniques; et

(c) de 0,1 à 10% en poids d'un agent tampon co-acide.

2. Procédé selon la revendication 1, dans lequel l'acide 2-hydroxyalcanoïque dans la composition est l'acide 2-hydroxyoctanoïque.

3. Procédé selon la revendication 1 ou 2, dans lequel l'acide 2-hydroxyalcanoïque dans la composition est présent en une quantité comprise entre 1 et 10% en poids.

4. Procédé selon l'une quelconque des revendications 1 à 3, dans lequel la composition contient 2 à 40% en poids de tensio-actif anionique.

5. Procédé selon la revendication 4, dans lequel le tensio-actif anionique est choisi parmi les acylglutamates, les acylpeptides, les sarcosinates, les esters d'acide carboxylique, les acyliséthionates, les α-oléfines sulfonates, les sulfosuccinates, les alkylsulfates, les alkyléthers sulfates, les alkylarylsulfonates, les amides de N-méthyltaurine ou les acides α-sulfo gras.

6. Procédé selon la revendication 4, dans lequel le tensio-actif anionique est choisi parmi le lauryléther sulfate de sodium 2OE ou le lauryle sulfate de sodium 3OE.

7. Procédé selon l'une quelconque des revendications précédentes, dans lequel l'agent tampon co-acide dans la composition est l'acide lactique.

8. Procédé selon l'une quelconque des revendications précédentes, dans lequel l'agent tampon co-acide dans la composition est présent en une quantité comprise entre 2 et 5% en poids.

9. Procédé selon l'une quelconque des revendications précédentes, dans lequel la composition comprend de plus un agent de dépôt en une quantité comprise entre 0,01 et 5% en poids.

10. Procédé selon la revendication 9, dans lequel l'agent de dépôt est choisi parmi le Polyquaternium 10, le Quaternium 40, le Quaternium 41 et le chlorure d'hydroxypropyltrimonium guar.

11. Procédé selon la revendication 9, dans lequel l'agent de dépôt dans la composition est présent en une quantité comprise entre 0,5 et 3% en poids.

12. Procédé selon l'une quelconque des revendications précédentes, dans lequel la totalité d'acide 2-hydroxyalcanoïque et d'agent tampon co-acide dans la composition est d'au moins 3% en poids de la composition.

13. Utilisation pour favoriser l'élasticité du cheveu d'un shampooing aqueux qui comprend:
    (a) de 0,1 à 20% en poids d'un acide 2-hydroxyalcanoïque choisi parmi l'acide 2-hydroxyhexanoïque, l'acide 2-hydroxyoctanoïque, l'acide 2-hydroxydécanoïque ou leurs mélanges;
    (b) de 2 à 40% en poids de tensio-actif choisis parmi des tensio-actifs anioniques, amphotériques et/ou zwittérioniques; et
    (c) de 0,1 à 10% en poids d'un agent tampon co-acide.